# EUROPEAN PATENT SPECIFICATION

(11) **EP 1 546 623 B1**
(45) Date of publication and mention of the grant of the patent: **22.04.2009**
(21) Application number: 03766300.2
(22) Date of filing: 25.07.2003
(51) Int. Cl.: F26B 3/36, A61L 11/00, B09B 3/00

(54) **PROCESS AND APPARATUS FOR COOKING AND STABILISATION OF BIO-ORGANIC MATERIALS**
VERFAHREN UND VORRICHTUNG ZUM ERHITZEN UND STABILISIEREN VON BIO-ORGANISCHEN MATERIALIEN
PROCEDE ET DISPOSITIF POUR CHAUFFER ET STABILISER DES MATIERES BIO-ORGANIQUES

(30) Priority: 29.07.2002 IT TO20020677
(43) Date of publication of application: 29.06.2005
(73) Proprietor: Officine Meccaniche Pejrani Srl, 10134 Torino (IT)
(72) Inventor: MORGANTINI, Gianpiero, I-10126 Torino (IT); PELLEGRIN, Ruggero, I-10131 Torino (IT)
(74) Representative: Rambelli, Paolo
(86) International application number: PCT/EP2003/008225
(87) International publication number: WO 2004/013554

(56) References cited:
- WO-A-01/02027
- WO-A-93/22059
- WO-A-95/03072
- WO-A-98/32548

## Description

The present invention relates to a process and to apparatus for a special cooking, by means of heat and locally generated steam, of material of biological nature, such as biomass, the rejects and refuse from food workings and, in general, materials which contain or can contain substances of biological nature, including living organisms, achieving stabilisation of the materials in the form of a dehydrated product, which is stable for long periods of time.

Non-limitative examples of materials of organic nature treatable with the process described here are grass cuttings, pruning residues, marine algae and seaweeds, slaughterhouse waste, vegetable peel and waste from food production, refuse from the food industry, agricultural activity or fishing, the organic fraction of solid urban waste, medical waste containing biological materials, and sludge from biological purifiers.

As is known, biomass materials and, in general, materials containing substances of organic and biological nature, are not stable. This is due to the presence of water and the other compounds in liquid phase which constitute, together with the organic substance, a substrate suitable for the development of fungi, seeds, insects and, in general, living organisms which are both contained in the material itself and which come from the environment in the form of pollen, spores, micro organisms etc. Their storage pending subsequent use or disposal often represents a problem, particularly in the case of materials of low economic value.

WO-A-9503072 discloses a process for the stabilization of bio-organic materials, such as natural biomass and refuse, such as grass cutting, pruning residue, marine algae and seaweed, slaughterhouse waste, peelings and vegetable waste from food production, food industry waste, waste from agricultural activity, fishing, the organic fraction of solid urban waste, medical waste containing biological material, sludge from biologic purifiers, such process comprising the operation of heating the particles of material by means of one or more movable members caused to move at high speed within a cylindrical casing having a vertical axis, close to fixed counteracting members in such a way to cause changes in volume constraining the material to compressions and deformations with the localized generation of a significant quantity of heat.

The process forming the subject of the present invention is intended for the transformation of such materials in such a way as to obtain a stable product completely free from liquids, which can be preserved for a long time without problems pending use, for example as agricultural fertilisers or manures, for animal feed, or whilst awaiting disposal.

The process forming the subject of the invention is characterised in that the material is first reduced to small particles, then subjected to repeated action of compression and deformation until reaching a high temperature losing all moisture, then heating and finally cooking by generation of steam from within the material itself by absorption of metered quantities of water. Peculiarities of the procedure are the operating conditions by which, by metering a controlled quantity of water onto the material heated to a temperature between 130 and 180°C, preferably about 160°C, this progressively penetrates in the form of a liquid into the depth of the granules of the material and is then transformed into steam at the expenses of the heat which is generated in the material by the mechanical effect.

The combined mechanical and thermal effects of the absorption of liquid water into the material undergoing treatment, followed by the transformation of the water into steam with a large change of volume, causes a slight lysis of the proteic materials and a mechanical action on the cell membrane. A perfectly dry and lifeless granular product free from spores, eggs, insects, seeds and living organisms in general is obtained from the treatment. The speed of the treatment makes it possible to obtain only a slight proteic lysis and therefore a good maintenance of the organaleptic properties of the material.

In comparison with other high temperature cooking treatments the process described here has the advantage of not requiring the use of pressure. Thus, it has advantages not only of simplicity of construction and management of the installations, but also in energy terms since pressure apparatus has the disadvantage of involving large masses of metal to heat and then cool at each treatment cycle. Finally, known pressure-cooking processes provide a treated material which is impregnated with water, which is not itself stable due to the water content. These treatments therefore involve the necessity of further treatments, such as the addition of preservatives or the use of ionising radiation, vacuum packaging etc.

By contrast, at the end of the treatment cycle the process forming the subject of the present invention provides a material which, as well as not containing live organisms, is stable due to its total desiccation.

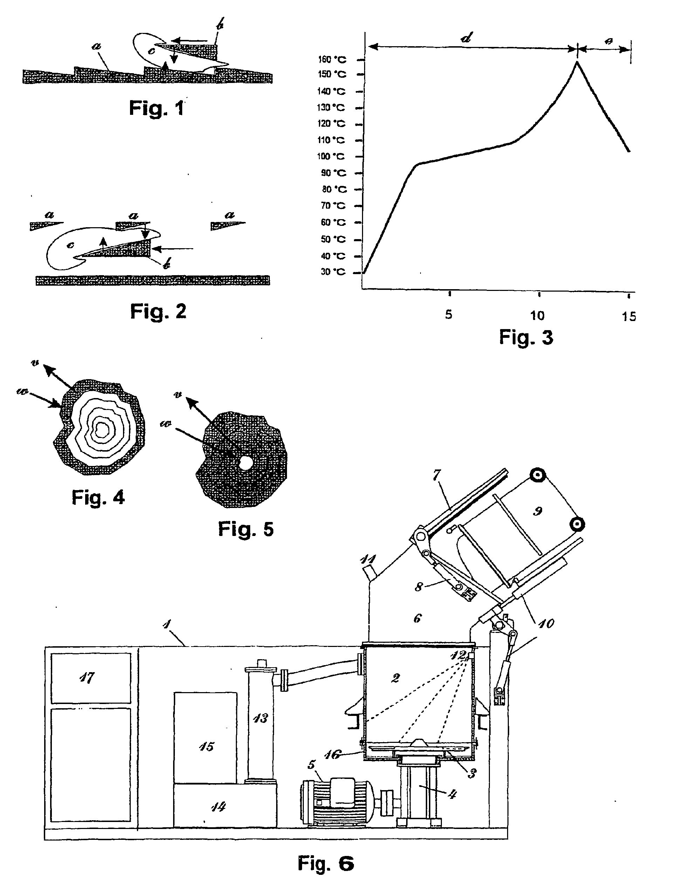

The compression actions which result in the formation of heat are exerted by means of a member which moves at high speed close to fixed members. One non-limitative example is that described in Figure 1 where the fixed members are represented by a band or strip in the form of a fixed circular crown provided with alternating projections and recesses or cavities, with a sawtooth transverse section, whilst the movable member is constituted by a rotating part having a *wedge-shaped* section passing a short distance from the toothed plane. In the arrangement described, the part with the fixed projections is formed on the wall which constitutes the bottom of a container. Alternatively, the fixed projections can be arranged on a side wall.

The material is forced by the movement of the movable *wedge-shaped* member into successive and repeated deformations which cause rapid heating of the material itself. As well as producing heat the movable member also produces a continuous mixing of the material.

In Figure 1 the letters (a) and (b) indicate, respectively, the fixed plane with its saw tooth projections and the movable member in the form of a wedge. The relative movement leads to formation of two compression forces in opposition which compress the particles of the material indicated with the letter (c), with deformations involving the formation of heat within the material itself. The high speed and repetition of the compression and deformation actions lead to the formation of a significant quantity of heat such as to heat the mass of the material, cause complete evaporation of liquids contained in it, further heating the material to a temperature greater than 100°C and to give off steam by the absorption of water in the superheated material.

Figure 2 shows a different arrangement for obtaining the compression effect where (a) indicates *wedge-shaped* projections fixed to the wall.

Figure 3 shows how the process develops. The graph plots the temperature of the material in degrees centigrade as a function of time in minutes. A heating and super heating phase indicated with the letter (d) having a duration of about 12 minutes is followed by a cooking phase indicated with (e) having a duration of about 3 minutes.

The material is initially heated from ambient temperature to a temperature of about 1000C. Subsequently, between 100 and 110°C the heat provided causes evaporation of all of the water and all the liquids present in the material. Then further heat provided causes superheating of the material.

Although the superheating temperature of the material could be taken beyond 180°C, limiting the superheating to 160°C avoids the commencement of thermal degradation of the organic substances.

It can be seen that the deformations due to the compressions generate heat throughout the mass of individual particles, directly within their interior, and therefore in a completely different manner from the typical heating by conduction effected by administration of heat from the outside. In this latter case the part of the particle at higher temperature will be the outer part, whilst in the process forming the subject of the present invention, on the contrary, it is the inner part which is at the higher temperature in that the heat which forms within it by effect of the deformation remains confined within the interior because of the thermal insulating nature of the material, until the intervention of the cooking phase consisting of absorption of water and formation of steam.

Once the superheating phase has been completed, the subsequent cooking phase is effected by causing the absorption of a controlled quantity of hot water by the material, still maintained in movement and being heated by the mechanical action of the movable member.

The added water is absorbed at the surface and then ever more into the interior of the particles from which heat is extracted by the generation of steam. Figure 4 schematically shows a particle of hot material during the commencement of the cooking phase. The letter (w) indicates the additional water and (v) represents the steam, which is emitted. Progressively, the surface layer of the particle in which the temperature has fallen sufficiently to permit the existence of liquid water gradually becomes deeper and deeper and this thus progressively penetrates and reaches by absorption the interior of the particle, developing steam ever further internally.

Figure 5 schematically shows a particle of material at the end of the cooking phase. This phase is interrupted when the super heated temperature of the mass has fallen to a little above 100°C; for example 105°C. The temperature of the particles still being greater than 100°C, these are perfectly dehydrated, dry and free running. The conditions of the operation result in a slight proteic lysis on the surfaces of the particles, rupture of the cellular membranes by internal pressure and coagulation of the cellular material invested by the steam, with devitalisation of seeds, eggs and micro organisms.

Further advantages and characteristics of the process and the apparatus according to the invention will become apparent from the following detailed description made with reference to the attached drawings, provided purely by way of non-limitative example, in which apparatus and some details for performance of the process are illustrated in a schematic manner.

Figure 6 shows the schematic arrangement of the apparatus. A frame (1) permits the assembly of components in such a way as to obtain a unitary apparatus. The main part of the machine is constituted by a casing (2) forming a chamber of cylindrical form with a vertical axis. This part, provided with a rotating member (3), constitutes the system for production of heat by compression and deformation of the material under treatment.

The rotating member is held in position by the support (4) and driven by the motor (5) at a speed of rotation between 1500 and 2500 revolutions per minute, in such a way as to have a peripheral speed of a rotor between 30 and 70 metres per second. The speed of rotation of the rotating member is controlled by a system, which constantly and automatically compares the speed at which the material during the various phase of the process becomes superheated with respect to the calibrated values.

The upper part of the casing connects to the loading inlet (6) provided with a cover (7) actuated by devices (8) of hydrodynamic type or equivalent. The material to be treated is introduced into the cell (2) by opening the cover (7) and tipping the contents of a container (9) by actuation of hydrodynamic devices (10) or the equivalent.

On the loading inlet (6) is installed an infrared temperature measuring device (11) facing downwardly in such a way as to measure the exact temperature of the material in real time. On the upper part of the casing (2) is arranged a nozzle (12) for dosing the cooking water. The dosing is controlled by an automatic logic which comes into operation and stops at the programmed temperatures.

The steam which is released from the casing during the process is conveyed by means of a duct to a system (13) for aspiration and extraction. This is constituted by a condenser operating by mixing steam with water. The condenser is connected with a tank (14) in which the separation of water from aspirated air is effected, which air is expelled after having been filtered in the filtration unit (15) comprising a droplet separator, a powder filter, an active carbon filter and an absolute filter.

The lower part of the casing (16) constituting the bottom is bolted on and replaceable in dependence on wear. An electrical control panel (17) completes the apparatus.

Figures 7, 8 and 9 respectively show a plan view and two sections (AA and BB) of the bottom (17) of the casing (2). Reference numeral (18) indicates the teeth of the bottom which can have a depth, indicated (19), of between 5 and 20mm.

Figures 10 to 11 show the section and plan of a construction arrangement where the compression-deformation system for the material is formed by movable wedges in co-operation with a toothed bottom. The movable member is constituted by the *wedge-shaped* elements (20) which are fixed to the blades (21) of the rotor (3). The rotor is provided with two or more blades.

Figure 12 shows the section of a constructional arrangement where the compression-deformation system for the material is obtained with movable wedges (20) in co-operation with *wedge-shaped* counter members (22) fixed to the side wall near the bottom (16) of the casing (2).

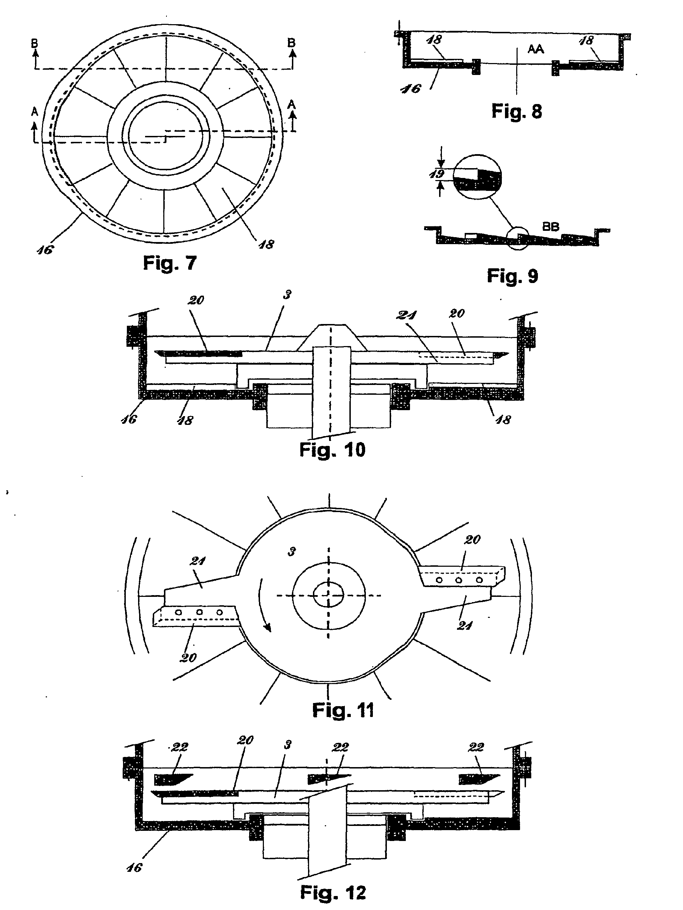

Figures 1, 2, 3, 4 and 5 show the principle of the process whilst Figures 5, 6, 7, 8, 9, 10, 11 and 12 show possible constructional arrangements which can be naturally modified with respect to the essential characteristics of the invention.

## Claims

1. A process for the stabilisation of bio-organic materials, such as natural biomass and refuse, such as grass cuttings, pruning residue, marine algae and seaweed, slaughterhouse waste, peelings and vegetable waste from food production, food industry waste, waste from agricultural activity, fishing, the organic fraction of solid urban waste, medical waste containing biological material, and sludge from biological purifiers, *such process comprising* the operation of heating the particles of material by means of one or more wedge-shaped members caused to move at high speed within a cylindrical casing having a vertical axis, close to fixed counteracting members in such a way as to cause changes in volume constraining the material to compressions and deformations with the localised generation of a significant quantity of heat said process being **characterized in that**:
- the fixed members and the movable members are wedge-shaped;
- the fixed members are located on a circular crown on an inner wall of the casing and have a transverse section defining a saw-tooth profile; and
- the movable members are suitable for compressing the bio-organic material towards the saw-tooth profile, so as to cause successive and repeated deformations of the bio-organic material across alternating projections and recesses of the saw-tooth profile.

2. A process according to Claim 1, in which the fixed members are formed as radial teeth in the bottom of the cylindrical casing.

3. A process according to Claim 1, in which the fixed members are formed as lateral teeth on the lower part of the cylindrical wall of the casing.

4. A process according to Claim 1, in which the movable members of the heating system are carried by a rotor provided with blades driven to rotate at high speed.

5. A process according to any preceding claim, in which the stabilisation and cooking treatment for the bio-organic materials is effected first by rendering them dry and then superheating the material to a temperature of between 130 and 180°C and then dosing it with water in such a way that this is absorbed progressively towards the interior of the dehydrated material from where it is emitted in the form of steam, leading to the conditions for devitalisation of seeds, spores, eggs and micro-organisms.

6. A process according to Claim 5, in which upon attainment of the programmed cooking temperature, the dosing of water is automatically controlled by a regulation system in such a way that the temperature of the material gradually falls but without passing beyond the lower limit of 100°C.

7. Apparatus intended for cooking and stabilisation of bio-organic materials, **characterised in that** it comprises:
- a cylindrical casing for containing the material;
- a rotor within the casing, provided with blades carrying *movable* members able to cause, in combination with fixed members on the bottom or walls of the casing, a restriction to the passage resulting in deformation and compression actions on the material, with a localised formation of heat able to bring the material rapidly to a temperature up to 180°C;
- infrared temperature measurement and control means positioned above the casing;
- means for emitting and dosing water, positioned in the upper part of the casing and controlled by the temperature measurement and control system;
- rotor means and systems for regulation of the speed operable to allow control of the quantity of heat produced; said apparatus being **characterized in that**:
- the fixed members and the movable members are wedge-shaped;
- the fixed members are located on a circular crown on an inner wall of the casing and have a transverse section defining a saw-tooth profile; and
- the movable members are suitable for compressing the bio-organic material towards the saw-tooth profile, so as to cause successive and repeated deformations of the bio-organic material across alternating projections and recesses of the saw-tooth profile.

8. Apparatus according to Claim 7, **characterised in that** it includes mechanised loading means for the material to be treated, means for condensation of the steam formed during the dehydration and water dosing phase into water, and filters for the treatment of the gases and vapours given off before discharge from the system.

## Patentansprüche

1. Verfahren zum Stabilisieren von bio-organischen Materialien, wie z.B. natürliche Biomasse und Abfall, wie z.B. Grasschnitt, Aufastreste, Meeresalgen und Seegras, Schlachtabfall, Schalen und Pflanzenabfall aus der Lebensmittelerzeugung, Lebensmittelindustrieabfall, Abfall aus landwirtschaftlichen Tätigkeiten, Fischen, der organische Anteil von Hausmüll, medizinischer Abfall, der biologische Materialien enthält, Schlicke von biologischen Reinigern, wobei ein derartiges Verfahren den Vorgang des Erhitzens der Materialpartikel unter Verwendung von einem oder mehreren keilförmigen Bauteilen aufweist, die dazu veranlasst werden, sich mit hoher Geschwindigkeit innerhalb eines eine vertikale Achse aufweisenden zylindrischen Gehäuses nahe an feststehenden entgegenwirkenden Bauteilen auf eine derartige Art und Weise zu bewegen, dass Volumenveränderungen verursacht werden, die das Material durch die lokale Erzeugung einer wesentlichen Hitzemenge zu Verdichtungen und Verformungen zwingen, wobei das Verfahren **dadurch gekennzeichnet ist, dass**:
- die feststehenden Bauteile und die beweglichen Bauteile keilförmig sind;
- die feststehenden Bauteile in einer kreisförmigen Krone an einer Innenwand des Gehäuses angeordnet sind und einen Querschnitt aufweisen, der ein Sägezahnprofil festlegt; und
die beweglichen Bauteile dazu geeignet sind, das bio-organische Material in Richtung des Sägezahnprofils zu verdichten, um sukzessive und wiederholte Verformungen des bio-organischen Materials über abwechselnde Vorsprünge und Aussparungen des Sägezahnprofils hinweg zu verursachen.

2. Verfahren nach Anspruch 1, bei dem die feststehenden Bauteile als Radialzähne in dem Boden des zylindrischen Gehäuses ausgebildet sind.

3. Verfahren nach Anspruch 1, bei dem die feststehenden Bauteile als Seitenzähne an dem unteren Teil der zylindrischen Wand des Gehäuses ausgebildet sind.

4. Verfahren nach Anspruch 1, bei dem die beweglichen Bauteile des Erhitzungssystems von einem Rotor getragen sind, der mit Flügeln versehen ist, welche angetrieben werden, um sich mit hoher Geschwindigkeit zu drehen.

5. Verfahren nach einem vorangehenden Anspruch, bei dem die Stabilisier- und Erhitzungsbehandlung für die bio-organischen Materialien dadurch bewirkt wird, dass diese zuerst getrocknet werden und anschließend das Material auf eine Temperatur zwischen 130 und 180°C überhitzt wird und dieses anschließend mit Wasser auf eine derartige Weise dosiert wird, dass dieses fortschreitend in Richtung des Inneren des entwässerten Materials absorbiert wird, von wo aus es in der Form von Dampf abgegeben wird, was zu den Gegebenheiten zur Devitalisation von Keimen, Sporen, Eiern und Mikroorganismen führt.

6. Verfahren nach Anspruch 5, bei dem nach Erreichen der programmierten Erhitzungstemperatur das Dosieren von Wasser automatisch durch ein Reguliersystem auf eine derartige Art und Weise gesteuert wird, dass die Temperatur des Materials allmählich abnimmt, aber ohne unter die untere Grenze von 100°C zu fallen.

7. Vorrichtung zum Erhitzen und Stabilisieren von bio-organischen Materialien, **dadurch gekennzeichnet, dass** es Folgendes aufweist:
ein zylindrisches Gehäuse zum Aufnehmen des Materials;
einen Rotor innerhalb des Gehäuses, der mit Flügeln versehen ist, die bewegliche Bauteile tragen, welche in der Lage sind, in Verbindung mit feststehenden Bauteilen an den Boden oder Wänden des Gehäuses eine Durchtrittsbeschränkung zu verursachen, was zu Verformungs- und Verdichtungsverhalten des Materials führt, zusammen mit einer lokalen Hitzeausbildung, die in der Lage ist, das Material schnell auf eine Temperatur bis zu 180°C zu bringen;
eine Infrarot-Temperaturmess- und Steuereinrichtung, die über dem Gehäuse positioniert ist;
eine Einrichtung zum Abgeben und Dosieren von Wasser, die in dem oberen Teil des Gehäuses positioniert ist und durch das Temperaturmess- und Steuersystem gesteuert wird;
eine Rotoreinrichtung und Systeme zum Regulieren der Geschwindigkeit, die betätigbar sind, um eine Steuerung der erzeugten Hitzemenge zu steuern;
wobei die Vorrichtung **dadurch gekennzeichnet ist, dass**
die feststehenden Bauteile und die beweglichen Bauteile keilförmig sind;
die feststehenden Bauteile in einer kreisförmigen Krone an einer Innenwand des Gehäuses angeordnet sind und einen Querschnitt aufweisen, der ein Sägezahnprofil festlegt; und
die beweglichen Bauteile geeignet sind, das bio-organische Material in Richtung des Sägezahnprofils zu verdichten, um sukzessive und wiederholte Verformungen des bio-organischen Materials über abwechselnde Vorsprünge und Aussparungen des Sägezahnprofils hinweg zu verursachen.

8. Vorrichtung nach Anspruch 7, **dadurch gekennzeichnet, dass** sie eine mechanisierte Ladeeinrichtung für das zu behandelnde Material, eine Einrichtung zum Kondensieren des während der Entwässerungs- und Wasserdossierphase ausgebildeten Dampfs zu Wasser und Filter für die Handhabung der ausgestoßenen Gase und Dämpfe aufweist, bevor diese aus dem System abgegeben werden.

## Revendications

1. Procédé pour la stabilisation des matières bio-organiques telles que la biomasse naturelle et les déchets tels que les coupes d'herbe, les résidus d'élagage, les algues marines et les algues, les déchets d'abattoir, les pelures et les déchets végétaux provenant de la production alimentaire, les déchets de l'industrie alimentaire, les déchets provenant de l'activité agricole, la pêche, la part organique des déchets urbains solides, des déchets médicaux contenant de la matière biologique, et les boues provenant des purificateurs biologiques, un tel procédé comprenant l'étape consistant à chauffer les particules de matière au moyen d'un ou de plusieurs éléments en forme de cale amenés à se déplacer à grande vitesse dans un boîtier cylindrique ayant un axe vertical, à proximité des éléments de neutralisation fixes de telle manière qu'ils provoquent des changements de volume, contraignant la matière à des compressions et des déformations avec la génération localisée d'une quantité significative de chaleur, ledit procédé étant **caractérisé en ce que** :
➢ les éléments fixes et les éléments mobiles sont en forme de cale ;
➢ les éléments fixes sont positionnés sur une couronne circulaire sur une paroi interne du boîtier et ont une section transversale définissant un profil en dents de scie ; et
➢ les éléments mobiles sont appropriés pour comprimer la matière bio-organique vers le profil en dent de scie, afin de provoquer des déformations successives et répétées de la matière bio-organique sur des saillies et des évidements alternés du profil en dents de scie.

2. Procédé selon la revendication 1, dans lequel les éléments fixes sont formés comme des dents radiales au fond du boîtier cylindrique.

3. Procédé selon la revendication 1, dans lequel les éléments fixes sont formés comme des dents latérales sur la partie inférieure de la paroi cylindrique du boîtier.

4. Procédé selon la revendication 1, dans lequel les éléments mobiles du système de chauffage sont supportés par un rotor prévu avec des lames entraînées pour tourner à grande vitesse.

5. Procédé selon l'une quelconque des revendications précédentes, dans lequel le traitement de stabilisation et de cuisson pour les matières bio-organiques est effectué dans un premier temps pour les sécher et ensuite surchauffer la matière à une température comprise entre 130 et 180°C et ensuite la doser avec l'eau de sorte que celle-ci est absorbée progressivement vers l'intérieur de la matière déshydratée à partir d'où elle est émise sous forme de vapeur, conduisant à des conditions de dévitalisation des graines, des spores, des oeufs et des micro-organismes.

6. Procédé selon la revendication 5, dans lequel suite à l'obtention de la température de cuisson programmée, le dosage de l'eau est automatiquement contrôlé par un système de régulation de sorte que la température de la matière chute progressivement mais sans passer au-delà de la limite inférieure de 100°C.

7. Appareil prévu pour la cuisson et la stabilisation de matières bio-organiques, **caractérisé en ce qu'**il comprend :
➢ un boîtier cylindrique pour contenir la matière ;
➢ un rotor à l'intérieur du boîtier, prévu avec des lames supportant des éléments mobiles pouvant provoquer, en combinaison avec des éléments fixes au fond ou sur les parois du boîtier, une restriction du passage, se traduisant par des actions de déformation et de compression de la matière, avec une formation localisée de chaleur pouvant amener rapidement la matière à une température allant jusqu'à 180°C ;
➢ des moyens de commande et de mesure de température infrarouges positionnés au-dessus du boîtier ;
➢ des moyens pour émettre et doser de l'eau, positionnés dans la partie supérieure du boîtier et commandés par le système de commande et de mesure de température ;
➢ des moyens de rotor et des systèmes pour le réglage de la vitesse fonctionnant pour permettre le contrôle de la quantité de chaleur produite ;
➢ ledit appareil étant **caractérisé en ce que** :
➢ les éléments fixes et les éléments mobiles sont en forme de cale ;
➢ les éléments fixes sont positionnés sur une couronne circulaire sur une paroi interne du boîtier et ont une section transversale définissant un profil en dents de scie ; et
➢ les éléments mobiles sont appropriés pour comprimer la matière bio-organique vers le profil en dents de scie, afin de provoquer des déformations successives et répétées de la matière bio-organique sur des saillies et des évidements alternés du profil en dents de scie.

8. Appareil selon la revendication 7, **caractérisé en ce qu'**il comprend des moyens de chargement mécanisés pour la matière à traiter, des moyens pour la condensation de la vapeur formée pendant la phase de dosage de l'eau et de déshydratation en eau, et des filtres pour le traitement des gaz et des vapeurs émis avant la décharge du système.
